# EUROPEAN PATENT APPLICATION

(11) **EP 3 563 703 A1**
(43) Date of publication of application: **06.11.2019**
(21) Application number: 16925540.3
(22) Date of filing: 28.12.2016
(51) Int. Cl.: A41D 13/00, A41H 3/00, A61B 5/11, G06F 1/16, G06F 1/18, G06F 3/01

(54) **WEARABLE DEVICE AND PATTERN PAPER**

(71) Applicant: Xenoma Inc., Tokyo 143-0013 (JP)
(72) Inventor: NAKAJIMA Masao, Tokyo 143-0013 (JP); AMIMORI Ichiro, Tokyo 143-0013 (JP)
(74) Representative: J A Kemp LLP
(86) International application number: PCT/JP2016/089152
(87) International publication number: WO 2018/123034

(57) **Abstract**

A wearable device 100 includes sensors 2, at least one of which being provided on the front side 1f and the back side 1b respectively, and a single controller 4 that controls the sensors 2. The boundary between the front side 1f and the back side 1b includes first seamed portions (13r, 131, 14r, and 141) and second seamless portions (11r, 111, 12r, and 121). Some of the wires 3 are disposed on the front side 1f and the back side 1b across the second seamless portions (11r, 111, 12r, and 121) on the boundary between the front side 1f and the back side 1b so as to electrically couple the sensors 2 provided on the front side 1f and the back side 1b to the controller 4.

## Description

### Technical Field

The present invention relates to wearable devices and paper patterns.

### Background Art

In recent years, research and development of stretchable electronics including stretchable wires and sensors have been advanced. For example, forming stretchable electronics on a garment and the like is drawing attention as it enables the garment to be used as a wearable device capable of obtaining information such as motions, of the wearer as well as biological information such as pulses, of the wearer.

As a method for forming such stretchable wires, there has been proposed a method of directly printing electrically conductive ink on a stretchable substrate (see Patent Literature 1) and a method of sewing or interweaving electrically conductive yarns into a stretchable substrate (see Patent Literature 2), for example.

Meanwhile, in smart apparel obtained by forming a circuit including electronic components such as sensors, actuators, and light sources, on a fabric, components that have the greatest difficulty in having flexibility are a power supply such as a lithium battery, a microcomputer including an A/D converter and the like, and a wireless communication module that uses Bluetooth (registered trademark) or Wi-Fi. Therefore, a method of coupling a controller, which is obtained by mounting the aforementioned components in a housing such as a plastic case, to a fabric via a connector is often used for smart apparel.

In efficiently producing such smart apparel, in order to control electronic components, which are disposed at a plurality of positions on the front and back sides of the body, with a single controller, it would be necessary to use circuits including wires and the like for coupling the electronic components.

### Citation List

### Patent Literature

[Patent Literature 1] JP 2016-130940 A
[Patent Literature 2] JP 2008-504856 A

### Summary of Invention

### Technical Problem

Conventionally, in order to produce smart apparel such as the one described above, different paper patterns are used for the front and back sides as with paper patterns for ordinary garments. Therefore, apparel that has been sewn in a three-dimensional shape in advance always has seamed portions on the boundary between the front and back sides thereof. Thus, when circuits are formed on such apparel, wires would be disposed across the seamed portions on the boundary between the front and back sides, which can result in breaking and damage of the wires. Otherwise, when circuits are formed such that their wires are not disposed across the boundary between the front and back sides, separate controllers would be needed to control the respective circuits on the front and back sides, which can result in increased cost and weight.

Meanwhile, when a printed circuit fabric (PCF), which has a circuit formed on a flat fabric, is formed, and the fabric is then sewn into the shape of a garment, similar problems also would arise.

The inventors have conducted concentrated studies to solve the aforementioned problems and as a result, conceived of utilizing, for producing smart apparel having sensors on both the front and back sides of the body, a special paper pattern in which the boundary between the front and back sides is continuous without any seams therebetween, to control the sensors on both the front and back sides using a single controller.

Specifically, the inventors produced a fabric in which the boundary between the front and back sides is partially continuous utilizing the aforementioned paper pattern, and conceived of a configuration in which wires are disposed on the front and back sides across the continuous portion on the boundary.

It is an object of the present invention to provide, based on the aforementioned finding of the inventors, a wearable device formed by disposing circuits on a garment, the wearable device having no wire for coupling sensors at positions across seamed portions of the garment, and the wearable device being controllable with a single controller and being capable of reducing possible damage to wires and also reducing cost and weight, as well as a paper pattern that can produce such a wearable device.

### Solution to Problem

(1) In order to solve the aforementioned problems, a wearable device according to an embodiment of the present invention is an upper-body wearable device having wires disposed on a fabric having a front side and a back side, the wearable device including electronic components, at least one of which being disposed on the front side and on the back side, respectively, and a single controller configured to control the electronic components. The boundary between the front and back sides of the fabric includes a first seamed portion and a second seamless portion. In addition, one or more of the wires are disposed on the front and back sides across the second seamless portion on the boundary so as to electrically couple the electronic components disposed on the front and back sides to the controller.
(2) In the wearable device of (1) according to another embodiment, each electronic component includes at least one of a sensor, an actuator, or a light source.
(3) In the wearable device of (1) or (2) according to another embodiment, each wire is a stretchable wire, the stretchable wire including a stretchable substrate and an electrically conductive yarn arranged in the stretchable substrate in a zigzag pattern.
(4) In the wearable device of any one of (1) to (3) according to another embodiment, one of the front side or the back side of the wearable device is further divided into a right half side and a left half side so as to allow the wearable device to be put on or off, and the controller is disposed on the right half side and the left half side across the divided portion therebetween.
(5) A paper pattern according to an embodiment of the present invention is a paper pattern for producing an upper-body wearable device, the wearable device including a fabric having a front side and a back side, wires disposed on the fabric, at least one electronic component disposed on each of the front and back sides, and a single controller configured to control the electronic components, one or more of the wires being disposed on the front and back sides across the boundary between the front and back sides so as to electrically couple the electronic components disposed on the front and back sides to the controller, in which at least a part of the paper pattern corresponding to the boundary between the front and back sides of the fabric is continuous.

### Advantageous Effects of Invention

The present invention with the aforementioned configurations can provide a wearable device that can, even when formed by disposing wires for coupling electronic components on a sewn garment, retain favorable wire characteristics without breaking or damage of the wires, which would otherwise occur if the wires are disposed on seamed portions of the garment, and in which the wires are controllable with a single controller, as well as a paper pattern that can produce such a wearable device.

Further, according to the aforementioned configuration of the present invention, there is no need to complicate the arrangement of wires to avoid breaking or damage of the wires due to seamed portions. Therefore, the wires can be easily controlled with a single controller.

### Brief Description of Drawings

Fig. 1A is a schematic plan view of the basic configuration of a wearable device according to an embodiment of the present invention, which illustrates the front side.
Fig. 1B is a schematic plan view of the basic configuration of a wearable device according to an embodiment of the present invention, which illustrates the back side.
Fig. 2 is a schematic plan view of the wearable device illustrated in Fig. 1, with a cover of a controller removed.
Fig. 3 is a schematic plan view of an example of a paper pattern used for producing the wearable device of the present invention.
Fig. 4 is a schematic plan view of another example of the same paper pattern.
Fig. 5 is a cross-sectional view of the configuration of a stretchable wire used in the present invention along line A-A' of Fig. 1A.
Fig. 6 is a cross-sectional view of the configuration of a stretchable wire used in the present invention along line B-B' of Fig. 1A.
Fig. 7A is a schematic view illustrating an electrically conductive yarn used in the present invention, specifically, a configuration in which a metallic yarn 33a and non-metallic yarn 33b are Z-twisted together.
Figs. 7B are schematic views illustrating electrically conductive yarns used in the present invention, specifically, exemplary configurations of various twist yarns.
Fig. 8 is a block diagram illustrating the configuration of a controller used for a sensor system used in the present invention.

### Description of Embodiments

Hereinafter, a wearable device according to embodiments of the present invention will be described in detail with reference to the accompanying drawings.

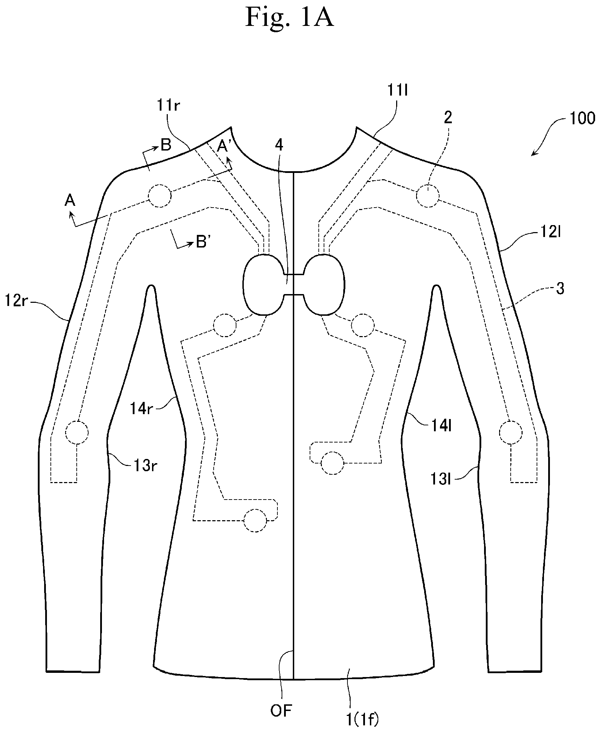

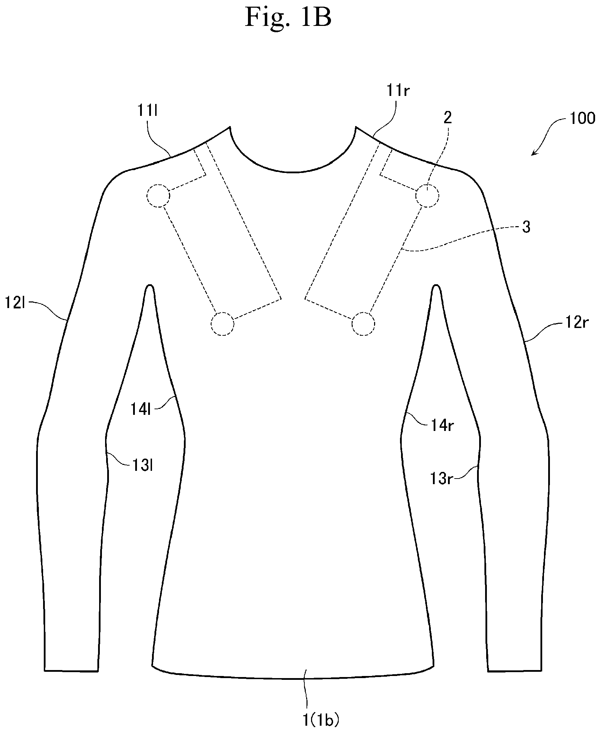

Figs. 1A and 1B are schematic plan views of the configuration of a wearable device 100 according to an embodiment of the present invention. Fig. 1A illustrates the front side and Fig. 1B illustrates the back side. As illustrated in Figs. 1A and 1B, the wearable device 100 according to this embodiment is the wearable device 100 for the upper body that is formed by disposing wires 3 on a fabric 1 having a front side If and a back side 1b. The wearable device 100 further includes at least one electronic component 2 (i.e., a sensor in this embodiment) on each of the front side 1f and the back side 1b, and a controller 4 that controls the sensors 2. Further, the boundary between the front side 1f and the back side 1b of the fabric 1 includes first seamed portions (13r, 131, 14r, and 141) and second seamless portions (11r, 111, 12r, and 121) (this point will be described later). Some of the wires 3 are disposed on the front side 1f and the back side 1b across the second seamless portions (11r, 111, 12r, and 121) on the boundary between the front side 1f and the back side 1b, so as to electrically couple the sensors 2 provided on the front side 1f and the back side 1b to the controller 4. It should be noted that the upper faces of the sensors 2 and the wires 3 are covered with protective layers, but the protective layers are not illustrated in Figs. 1A and 1B to help easily understand the sensors 2 and the wires 3.

Herein, "fabrics" as referred to in this specification mean garments and the like that may be put on the upper body, that is, a garment including a front side and a back side. Specifically, fabrics refer to garments with long sleeves, with three-quarter sleeves, with short sleeves, or without sleeves, or tank tops. This embodiment will describe a case where the fabric 1 is a garment with long sleeves.

Such garments can be produced with various materials used for ordinary garments. For example, natural fibers such as cotton, hemp, wool; chemical fibers such as polyester, nylon, or acrylic; or mixed fibers of such materials can be used. It should be noted that the fabric 1 forming a garment is preferably in as close contact as possible with the wearer so that motions of the wearer can be detected with high sensitivity. To this end, the fabric 1 is preferably made of a stretchable material using elastic fibers such as polyurethane.

The fabric 1 has plural sensors 2. At least one sensor 2 is disposed on the front side 1f and on the back side 1b, respectively. The sensors 2 are disposed on the fabric 1 at positions corresponding to the shoulders, elbows, back, abdomen, and the like to detect motions of the wearer. The number and positions of the sensors 2 are appropriately selected in accordance with the intended use of the wearable device 100.

For each sensor 2, a photodiode, a temperature sensor, a strain sensor, a pressure sensor, or an accelerometer can be used, for example. In addition, different sensors 2 may be used at different positions.

The sensor 2 is not particularly limited to a specific one as long as the amount of current flowing through the sensor 2, voltage applied to the sensor 2, the resistance of the sensor 2, and/or the capacitance of the sensor 2, for example, will change in response to changes in the physical quantity detected. Ideally, a variable-resistance sensor whose resistance and voltage applied across the sensor will change in response to changes in the physical quantity detected is used for the sensor 2 from perspectives of simplicity of the circuit and the like. Preferably, a digital sensor is used in response to demand for high-speed communication such as I2C. As the physical quantity, at least one of sound, light, temperature, or pressure can be favorably used. In such a case, the resistance value of the sensor 2 is preferably 50 times or more that of the wire 3.

In addition, a sensor that uses ink is preferably used for the sensor 2. A sensor that uses ink is a sensor produced with ink that has been obtained by mixing electrically conductive particles in an elastomer solution or a dispersed material, for example. Printing such ink and drying it can provide a sensor in which the electrically conductive particles are randomly dispersed in the elastomer film. Resistance across such a sensor will change as the distances between the electrically conductive particles change in response to tension applied, compression, or expansion or shrinkage due to a temperature change. A sensor that uses ink is very thin and has high trackability with respect to a target to be measured. Therefore, accurate and stable measurement is possible.

The plurality of sensors 2 are coupled by the wires 3. Each wire 3 includes, as described below, a wire layer formed by sewing an electrically conductive yarn into a stretchable substrate, and a short-circuit prevention layer provided between the fabric 1 and the wire layer.

The wires 3 coupled to the sensors 2 are coupled to the controller 4. The controller 4 includes, as described below, connector portions coupled to the wires 3, and a circuit board having various circuits mounted thereon. With the functions of such circuits, the controller 4 gathers information detected by the sensors 2, and sends the information to the outside or records it on a recording medium.

In this embodiment, as illustrated in Figs. 1A and 1B, some of the wires 3 are disposed on the front side 1f and the back side 1b across the seamless shoulder portions (11r and 111) of the fabric 1. This means that the shoulder portions can be produced using a paper pattern that is continuous without any seams as described below.

In this embodiment, the front side 1f of the fabric 1 has a front open portion OF formed therein. A zipper, buttons, or the like is formed on the front open portion so that the wearable device 100 can be put on or off as the front open portion OP is opened or closed. The wires 3 are disposed while avoiding the front open portion OP.

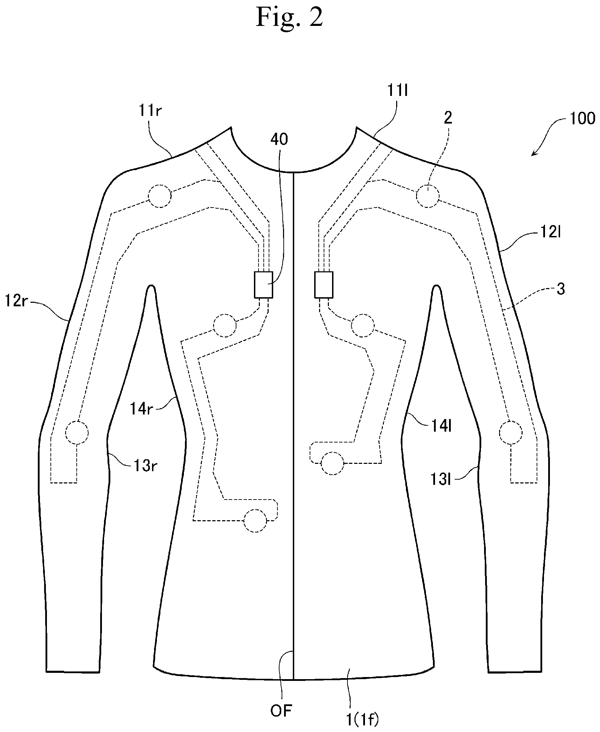

Fig. 2 illustrates the wearable device 100 according to this embodiment, with a cover of the controller 4 removed. As illustrated in Fig. 2, in this embodiment, the controller 4 includes two connectors 40. The wires 3 disposed on the right half side of the wearable device 100 and the wires 3 disposed on the left half side of the wearable device 100 are coupled to the two connectors 40, respectively.

The distance between the two connectors 40 is preferably less than or equal to 30 cm. If the distance between the two connectors 40 is too long, the size of the cover for the connector 40 becomes large, which can result in increased weight and deteriorated balance. In this embodiment, the cover for the two connectors 40 includes two circular portions that cover the respective connectors 40 and a coupling portion coupling the circular portions together. However, needless to say, the shape of the controller 4 is not limited thereto and can be other shapes such as a rectangular shape and an elliptical shape.

As described above, in the wearable device 100 according to this embodiment, the fabric 1 includes the first seamed portions (13r, 131, 14r, and 141) and the second seamless portions (11r, 111, 12r, and 121). More specifically, the flank side faces (14r and 141) and the inner side of the arms (13r and 131) of the fabric 1 are sewn. Meanwhile, the shoulder portions (11r and 111) and the outer sides of the arms (12r and 121) are not sewn.

The wires 3 are disposed on the seamless shoulder portions (11r and 111) at positions where the wires 3 span across the boundary between the front side 1f and the back side 1b of the fabric 1. As the wires 3 are disposed across the second seamless portions in this manner, breaking and damage of the wires due to the seamed portions can be prevented, and thus, degradation of the transmission properties of the wires can be suppressed.

Further, since the wires 3 can be disposed across the boundary between the front side 1f and back side 1b via the second seamless portions, there is no need to separately control the wires 3 disposed on the front side 1f and the back side 1b, and thus, the wires 3 can be collectively controlled with a single controller 4. Accordingly, advantageous effects are provided in that control stability can be improved, an increase in weight can be suppressed, and cost can be reduced.

Although the wires 3 are disposed across the shoulder portions (11r and 111) in this embodiment, the wires 3 may also be disposed across the boundary between the front side 1f and the back side 1b via the outer sides of the arms (12r and 121) that are the other seamless portions.

Although this embodiment illustrates a configuration in which the sensors 2 are disposed on the fabric 1, actuators or light sources such as LEDs, may be disposed in addition to or instead of the sensors 2. Alternatively, the controller 4 may be provided with such functions.

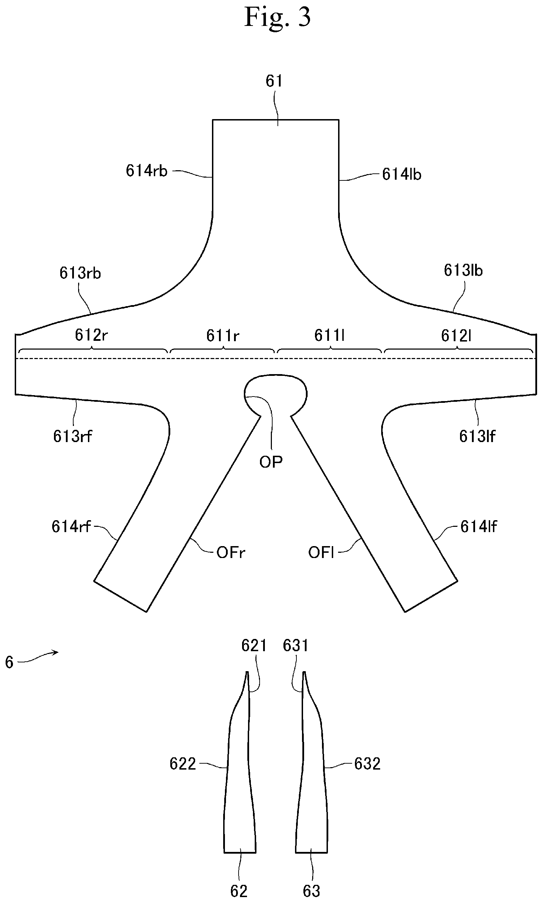

Fig. 3 is a plan view of a paper pattern 6 used for producing the fabric 1 of the wearable device 100 according to this embodiment. As illustrated in Fig. 3, the paper pattern 6 according to this embodiment includes a paper pattern body 61 corresponding to the front side 1f and the back side 1b of the fabric 1, and a pair of paper pattern flank portions 62 and 63 that are sewn to the respective side faces of the flanks of the portion 61 so as to secure three-dimensional appearance of the garment.

As illustrated in Fig. 3, paper pattern portions (6111 and 611r) of the body 61 corresponding to the shoulder portions (111 and 11r) of the fabric 1, and paper pattern portions (6121 and 612r) of the body 61 corresponding to the outer side portions of the arms (121 and 12r) of the fabric 1 are integrally formed, and thus, no sewing is needed. Meanwhile, paper pattern portions (613lf, 613lb, 613rf, and 613rb) corresponding to the inner sides of the arms (131 and 13r) of the fabric 1, and paper pattern portions (614lf, 614lb, 614rf, and 614rb) corresponding to the flank side faces (14l and 14r) of the fabric 1 are isolated.

After a fabric is cut along the paper pattern 6, portions (613lf and 613rf) on the front side and portions (613lb and 613rb) on the back side of the portions (613lf, 613lb, 613rf, and 613rb) are sewn together, respectively, so as to form the inner sides of the arms (131, 13r) of the fabric 1.

In addition, the front side portions (614lf, 614rf) of the portions (614lf, 614lb, 614rf, and 614rb) and the front side portions (632, 622) of the flank portions (63 and 62) are sewn together, respectively. The back side portions (614lb and 614rb) and the back side portions (631 and 621) of the flank portions (63 and 62) are sewn together, respectively This forms the flank side faces (141 and 14r) of the fabric 1. Then, a zipper, buttons, or like is formed on the left front open portion OFl and the right front open portion OFr so that the fabric 1 is obtained. It should be noted that reference symbol OP is the portion to become an opening for passing the neck of the wearer after sewing is finished.

Using such a paper pattern can produce the fabric 1 with seamless shoulder portions as described above. That is, using such a paper pattern can realize the wearable device 100 according to this embodiment and prevent breaking and damage of the wires as described above. Further, as is obvious from the configuration of the paper pattern 6 and the above description, the production of the fabric 1 and the arrangement of the wires 3 may be performed in any order, thus allowing for flexible process design.

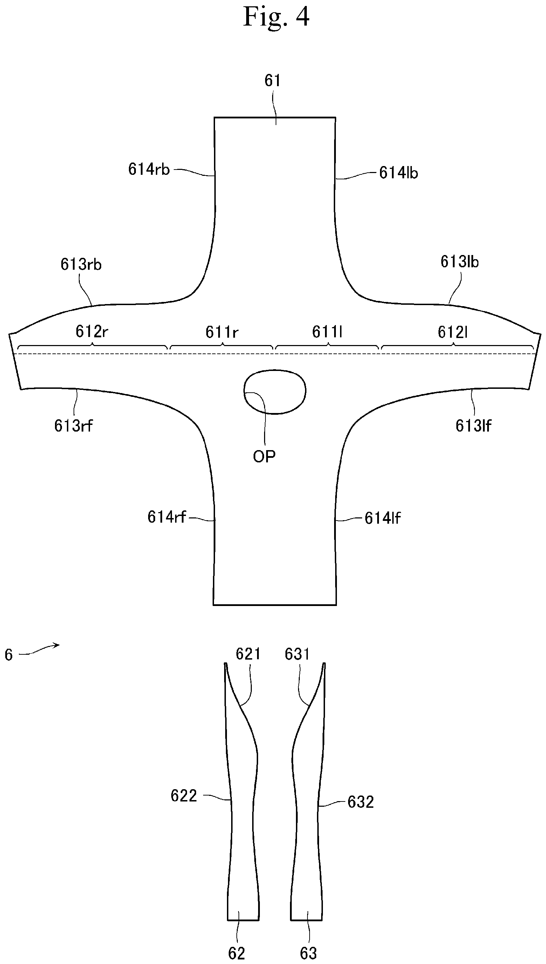

Fig. 4 is a plan view of the configuration of a paper pattern according to another embodiment. The paper pattern illustrated in Fig. 4 differs from that illustrated in Fig. 3 in that the pattern is not divided on either the front side or the back side. When the fabric 1 is produced using such a paper pattern, a so-called pullover wearable device, which is adapted to be put on by moving the head of the wearer through the device, is obtained. In such a case, since the front side is not divided in half unlike the fabric 1 produced using the paper pattern of Fig. 3, flexibility of the arrangement of the wires 3 on the front side can be increased.

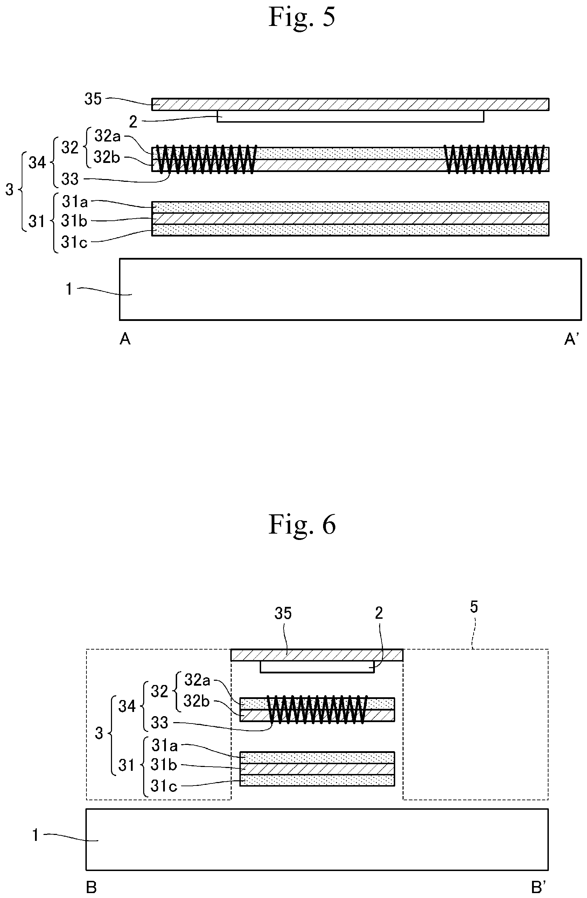

Figs. 5 and 6 are exemplary cross-sectional views of a portion including the sensor 2 of the wearable device 100 illustrated in Fig. 1 along line A-A' and line B-B', respectively. As illustrated in Figs. 5 and 6, the wire 3 includes a stacked structure of a short-circuit prevention layer 31 and a wire layer 34 provided on the fabric 1. In this embodiment, the sensor 2 is provided on the wire layer 34. Further, a protective layer 35 is provided so as to cover the upper face of the wire layer 34 and the upper face of the sensor 2. In Figs. 5 and 6, the fabric 1 and the short-circuit prevention layer 31; the short-circuit prevention layer 31 and the wire layer 34; and the wire layer 34 and the sensor 2 are illustrated as being partially isolated from each other to help understand the stacked structure of the layers and the like, but they are actually in contact with each other.

The wire layer 34 includes a stretchable film 32 and an electrically conductive yarn 33 sewn into the stretchable film 32 in a zigzag pattern. The stretchable film 32 includes a bonding layer 32a and an insulating layer 32b. The bonding layer 32a is preferably a thermoplastic bonding layer. Examples of the thermoplastic bonding layer include a known hot-melt film. Preferably, the thermoplastic bonding layer is a stretchable hot-melt film. Accordingly, the trackability of the wire layer 34 with respect to the fabric 1 when the fabric 1 is stretched or contracted improves. Examples of the stretchable hot-melt film include a hot-melt film containing polyurethane as a main component. In addition, as the insulating layer 32b, a stretchable insulating film such as a polyurethane film, can be used.

As described above, in this embodiment, the electrically conductive yarn 33 is sewn into the stretchable film 32 in a zigzag pattern. Herein, "zigzag" as referred to in this embodiment means, as illustrated in Figs. 5 and 6, that the electrically conductive yarn 33 is arranged such that it is directed both to a plane of the stretchable film 32 facing the sensor 2, and to another plane of the stretchable film 32 facing the short-circuit prevention layer 31, in an alternate manner. Although this embodiment illustrates a case where the electrically conductive yarn 33 is sewn, it may also be arranged in a zigzag pattern using other methods such as embroidery, knitting, or weaving.

The electrically conductive yarn 33 is exposed at least on the plane of the stretchable film 32 facing the sensor 2. Such a configuration can obtain an electrical connection between the electrically conductive yarn 33 and the sensor 2. Although the electrically conductive yarn 33 of this embodiment is also exposed on the plane of the stretchable film 32 facing the short-circuit prevention layer 31, the electrically conductive yarn 33 need not necessarily be exposed on this side.

In this embodiment, a twist yarn that uses a metal wire not covered with an insulating layer is used for the electrically conductive yarn 33. That is, metal of a metal wire used for the electrically conductive yarn 33 is exposed on its outer peripheral face. Using such a twist yarn for the electrically conductive yarn 33 can reduce resistance in comparison with a conventionally used yarn, which is formed by plating the surface of compound fibers with metal such as silver and thus has electrical conductivity imparted thereto, by about double digits. Further, in comparison with a coated metal wire, a significant decrease in electrical conductivity due to partial breaking can be prevented. The specific configuration of the twist yarn for the electrically conductive yarn 33 will be described later.

Further, the electrically conductive yarn 33 is preferably formed in a wave shape as seen from a direction parallel with the surface of the stretchable film 32 (i.e., a direction perpendicular to the plane of the paper). Forming the electrically conductive yarn 33 in a wave shape can secure a large margin for deformation (stretching) of the electrically conductive yarn 33 from the wave shape into a shape closer to a linear shape as it is stretched in the horizontal direction. This can increase the trackability of the electrically conductive yarn 33 in response to stretching of the stretchable film 32. In addition to the wave shape, the electrically conductive yarn 33 can also have various other shapes such as serpentine, zigzag, and horseshoe shapes.

As described above, when each of the bonding layer 32a and the insulating layer 32b is formed as a stretchable layer, the stretchable film 32 becomes stretchable. Further, sewing the electrically conductive yarn 33 into the stretchable film 32 in a wave pattern can obtain the wire layer 34 with high stretchability. Therefore, the wearable device 100 capable of tracking deformation such as stretch and flexure, that occurs in response to motions of the wearer can be obtained. Herein, the stretchability of the wire layer 34 is such that the acceptable amount of change in the shape of the wire layer 34 from the initial shape is preferably greater than or equal to 30%, more preferably, greater than or equal to 50%, and particularly preferably, greater than or equal to 100%. . Further, when the difference between the stretchability of the fabric 1 and/or the stretchability of the sensor 2 is greater than or equal to a given amount, stretch of each member would be disturbed or the detection sensitivity of the sensor would decrease. Therefore, the difference between the stretchability of the fabric 1 and the stretchability of the sensor 2 is preferably within a given range.

The thickness of the stretchable film 32 is not particularly limited, but it is preferably 5 to 300 µm, or more preferably, 10 to 100 µm. The stretchable film 32 in such a thickness range can achieve both high stretchability and high strength.

The stretchable film 32 is not limited to the aforementioned configuration. For example, the stretchable film 32 may include only the insulating layer 32b made of a polyurethane film, or the insulating layer 32b may have bonding layers formed on opposite faces thereof. Alternatively, the stretchable film 32 may include only the bonding layer 32a.

In Figs. 5 and 6, the wire layer 34 and the sensor 2 are in direct contact with each other, and the electrically conductive yarn 33 is exposed on the plane of the wire layer 34 facing the sensor 2. Therefore, the sensor 2 and the electrically conductive yarn 33 are in direct contact with each other. However, an electrically conductive hot-melt film containing electrically conductive metal particles may be added to the portion where the sensor 2 and the electrically conductive yarn 33 are in contact with each other. This can strengthen the electrical connection between the sensor 2 and the electrically conductive yarn 33 and reduce contact resistance. Electrically conductive fine metal particles used for the electrically conductive hot-melt film are preferably silver, gold, copper, platinum, or aluminum. Among them, silver, gold, copper are more preferable, and silver is the most preferable. Further, using flaky fine metal particles can impart high electrical conductivity even when the amount of the particles is small. The electrically conductive hot-melt film preferably includes a hot-melt film containing polyurethane as a main component. This can suppress peeling of the hot-melt film off the sensor 2 or the electrically conductive yarn 33 due to distortion when the stretchable substrate 1 is stretched.

In this embodiment, the short-circuit prevention layer 31 is provided between the wire layer 34 and the fabric 1. The short-circuit prevention layer 31 has a configuration in which the bonding layer 31a, the insulating layer 31b, and the bonding layer 31c are stacked in this order from the side of the wire layer 34. The short-circuit prevention layer 31 is provided for purposes of insulation and protection of the face of the wire layer 34 on the side of the stretchable substrate 1.

The insulating layer 31b that secures the insulation property of the short-circuit prevention layer 31 is formed of an insulating film or the like. Providing the insulating layer 31b allows the electrically conductive yarn 33, which is exposed on the plane of the wire layer 34 on the side of the stretchable substrate 1, to be embedded and can secure the insulating property of the wire layer 34 on the side of the stretchable substrate 1. This can suppress short-circuit of the wearable device 100 due to sweat and the like, and also allows the wearable device 100 to be used in water and the like. Further, providing the short-circuit prevention layer 31 can further increase the strength of the entire wire 3. As the insulating film forming the insulating layer 31b, a polyurethane film is preferably used, for example. This can secure a high insulation property and improve the trackability of the short-circuit prevention layer 31 when the stretchable substrate 1 is stretched.

In addition, the short-circuit prevention layer 31 includes the bonding layers 31a and 31c provided on opposite faces of the insulating layer 31b. With the bonding layers 31a and 31c provided, the short-circuit prevention layer 31 also has a function of bonding the stretchable substrate 1 and the wire layer 34 together.

For the bonding layers 31a and 31c, thermoplastic bonding layers are preferably used. Examples of the thermoplastic bonding layers include known hot-melt films, but stretchable hot-melt films are preferably used. Examples of the stretchable hot-melt films include a hot-melt film containing polyurethane as a main component. Accordingly, the trackability of the bonding layers when the stretchable substrate 1 is stretched improves, and the stretchable substrate 1 and the wire layer 34 can be firmly bonded together. Further, since such a configuration allows the stretchable substrate 1 and the wire layer 34 to be firmly bonded together, it is possible to prevent the short-circuit prevention layer 31 from peeling off the stretchable substrate 1 even when the wearable device 100 is subjected to treatment with water such as laundry.

Although Figs. 5 and 6 illustrate a case where the electrically conductive yarn 33 of the wire layer 34 is exposed on the plane of the stretchable film 32 on the side of the stretchable substrate 1, the electrically conductive yarn 33 may not be exposed on the plane of the stretchable film 32 on the side of the stretchable substrate 1 in some cases, as described above. In such a case, the plane of the stretchable film 32 on the side of the stretchable substrate 1 corresponds to the insulating layer 32b made of an insulating film, and a high insulation property is already secured. Thus, the short-circuit prevention layer 31 in such a case may include only a bonding layer without the insulating layer 31b. In addition, the short-circuit prevention layer 31 has a function of bonding the stretchable substrate 1 and the wire layer 34 together.

The thickness of the short-circuit prevention layer 31 is not particularly limited, but it is preferably 10 to 800 µm, or more preferably, 30 to 300 µm. As the structure of each layer of the short-circuit prevention layer 31, the thickness of the insulating layer 31b is preferably 5 to 300 µm, or more preferably, 10 to 100 µm. The insulating layer 31b in such a thickness range can maintain high stretchability and high strength. The thickness of each of the bonding layers 31a and 31c is preferably 10 to 200 µm, or more preferably, 30 to 100 µm. The bonding layer 31c in such a thickness range, in particular, can secure a sufficient bonding strength between the fabric 1 and the wire layer 34.

The short-circuit prevention layer 31 can have various configurations other than the aforementioned configuration. For example, short-circuit prevention layer 31 may include a plurality of different types of insulating layers.

The aforementioned stacked structure of the short-circuit prevention layer 31 and the wire layer 34 form the wire 3. The electric resistance of the wire 3 is preferably 0.005 to 0.5 Q/cm, more preferably, 0.005 to 0.2 Q/cm, or further preferably, 0.005 to 0.1 Q/cm. In addition, the rate of change of the resistance of the wire 3 when it is stretched from the initial state by 50% is preferably less than or equal to 5% of the resistance of the wire 3 before it is stretched.

The sensor 2 is disposed on the upper face of the wire layer 34 that is the upper layer portion of the wire layer 3, and further, the protective layer 35 is provided on the upper face of the sensor 2. A face of the protective layer 35 on the side opposite to the sensor 2 is the uppermost face when the wearable device 100 is used. That is, covering the wire layer 34 and the sensor 2 with the protective layer 35 can shield the surface of the sensor 2 and the wire layer 34 from the outside air. In this embodiment, the protective layer 35 has an insulating property. Therefore, the sensor 2 and the wire 3 can be protected from erosion due to sweat and the like when the wearable device 100 is used.

The protective layer 35 is the portion that becomes the uppermost face when used as the wearable device as described above. Therefore, the protective layer 35 preferably has stretchability as well as an insulating property. Specifically, the protective layer 35 is preferably a layer containing polyurethane, for example.

The thickness of the protective layer 35 is preferably 5 to 300 µm, or more preferably, 10 to 100 µm. The protective layer 35 in such a thickness range can maintain high stretchability and high strength.

As illustrated in Fig. 3, the protective layer 35 and the wire 3 (the short-circuit prevention layer 31 and the wire layer 34) are preferably provided only on the minimum required portion that covers the upper face of the sensor 2. With such a configuration, a space 5 having nothing provided therein is formed above the stretchable substrate 1. Providing such a space 5 can further increase the air permeability and flexibility of the wearable device 100.

Herein, the minimum required portion that covers the upper face of the sensor 2 is preferably in the range of 0.1 to 100 mm, or more preferably, 0.5 to 5 mm from the outer periphery of the sensor 2 and the wire 3 as seen in plan view. A too large portion covering the upper face of the sensor 2 would increase unnecessary portions and disturb the air permeability of the wearable device 100. Meanwhile, a too narrow portion covering the upper face of the sensor 2 would increase the possibility that the sensor 2 and wire 3 will be partially exposed to the outside due to steps between the sensor 2 and the wire 3, thus increasing the possibility of short-circuit.

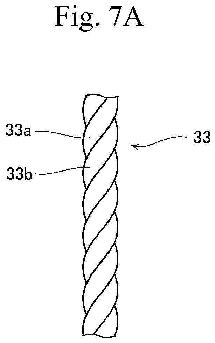

Next, the electrically conductive yarn 33 according to an embodiment of the present invention will be described with reference to Fig. 7A. The electrically conductive yarn 33 used in this embodiment is formed by twisting a single twist metallic yarn 33a and a single twist non-metallic yarn 33b (i.e., natural fibers or chemical fibers), which is commonly used, together as illustrated in Fig. 7A. The electrically conductive yarn 33 may be not only a plied yarn formed by twisting single twist yarns together but also a corkscrew twist yarn formed by combining a single twist yarn and a non-twist yarn, a covering having a yarn wound on its outermost layer, or a yarn formed by repeatedly twisting yarns together. In addition, the outermost layer, which is the final twist yarn layer, of the electrically conductive yarn 33 is preferably a Z-twist yarn layer. This is because the electrically conductive yarn 33 is often sewn with a sewing machine when used for the wearable device 100.

When a yarn formed by plating the surface of a non-metallic yarn with metal is used as the electrically conductive yarn as is conventional, the conductive portion of the electrically conductive yarn is only the metal plated portion covering the surface of the electrically conductive yarn. That is, the non-metallic yarn with quite high resistivity accounts for a large portion of the cross-sectional area of the electrically conductive yarn, thus increasing the resistivity of the entire electrically conductive yarn. Meanwhile, if a yarn formed by twisting the metallic yarn 33a and the non-metallic yarn 33b together is used as the electrically conductive yarn 33 as in this embodiment, provided that the thickness of the metallic yarn 33a is about equal to the cross-sectional area of the non-metallic yarn 33b, the metallic yarn 33a accounts for about a half of the cross-sectional area of the electrically conductive yarn 33. Thus, the resistivity of the electrically conductive yarn 33 becomes quite low. Therefore, the transmission speed of signals through the wire significantly improves, and a high-quality wire can be obtained. Naturally, the resistivity of the metallic yarn 33a should also be in a sufficiently low resistivity range.

The electrically conductive yarn 33 stretches as the stretchable wire 32 stretches. However, if the metallic yarn 33a partially forming the electrically conductive yarn 33 breaks when the fabric 1 is greatly stretched, the function of the wire would be lost. Thus, the metallic yarn 33a should be prevented from breaking when the stretchable wire 32 stretches. Specifically, the breaking elongation of the metallic yarn 33a should exhibit a value greater than or equal to a given value. The "breaking elongation" is a value obtained by, in a tensile test for a given metallic material, expressing the permanent elongation of a test piece after it breaks in percentage relative to the original marked distance.

The electrically conductive yarn 33 is sewn into the stretchable film 32 as described above. Herein, if the stretchable film 32 is sewn with a sewing machine using a thick needle like a commonly used sewing needle, holes formed by the needle may be large, possibly breaking the film. Therefore, the electrically conductive yarn 33 should be thin enough to prevent the stretchable film 32 from breaking. Specifically, the non-metallic yarn 33b is preferably as thin as 10 to 150 deniers and sufficiently strong.

As described above, the metallic yarn 33a used for the electrically conductive yarn 33 should have at least electrical conductivity (or resistivity) satisfying given conditions. Further, the yarn is selected taking into consideration various other conditions such as workability, cost, and durability. Table 1 shows the resistivity of various metals that can be used for the wire. "Evaluation" in the table indicates if each metal has suitable resistivity to be used as a wire, and "Excellent" means that the metal has highly suitable resistivity to be used as a wire, "Good" means that the metal has suitable resistivity to be used as a wire, and "Acceptable" means that the metal has acceptable resistivity to be used as a wire, and "Unacceptable" means the metal does not have acceptable resistivity to be used as a wire.

**[Table 1]**

| Name of Metal | Resistivity | Evaluation |
|---|---|---|
| | (× 10⁻⁶ Ω·cm) | |
| Aluminum | 2.655 | Good |
| Titanium | 42 | Unacceptable |
| Chromium | 12.9 | Unacceptable |
| Iron | 9.71 | Unacceptable |
| Nickel | 6.84 | Unacceptable |
| Copper | 1.67 | Excellent |
| Silver | 1.59 | Excellent |
| Tungsten | 5.65 | Acceptable |
| Platinum | 10.6 | Unacceptable |
| Gold | 2.35 | Good |

As shown in the table above, aluminum, copper, silver, tungsten, or gold is metal with resistivity that can be used for a metal wire to form the metallic yarn 33a. Further, copper is particularly preferably used, taking into consideration the aforementioned workability, cost, and durability altogether. Further, alloys of such metals can also be used. When an alloy is used, its resistivity is preferably in the "Acceptable" range, more preferably in the "Good" range, and most preferably in the "Excellent" range. Specifically, the resistivity is preferably less than or equal to 5.00 (× 10⁻⁶ Ω·cm), more preferably, less than or equal to 3.00 (10⁻⁶ Ω·cm), or further preferably, 2.00 (× 10⁻⁶ Ω·cm). Specific examples of alloys include beryllium copper, zirconium copper, brass, bronze, phosphor bronze, titanium copper, and cupro-nickel. In addition, such metal wires can be plated. Specific examples of metals used for plating include tin, zinc, copper, silver, nickel, aluminum, titanium, platinum, gold, and alloys thereof.

The thickness of the metal wire forming the metallic yarn 33a is preferably 0.01 to 0.20 mm, or more preferably, 0.02 to 0.10 mm. In particular, when a copper wire is used as a metal wire to form the metallic yarn 33a, the thickness thereof is quite preferably 0.025 to 0.08 mm.

For the non-metallic yarn 33b, natural fibers, chemical fibers, and the like that have been conventionally used can be used, but a polyurethane yarn or a polyester yarn is particularly preferably used. The thickness of the non-metallic yarn 33b is preferably 10 to 150 deniers.

As the number of twists of the electrically conductive yarn 33 is greater, the length of the metallic yarn 33a per unit length becomes greater. Therefore, a load that acts on the metallic yarn when it stretches can be reduced, and thus, metal fatigue can be suppressed. The specific number of twists is preferably 100 to 450 T/m, or more preferably, 200 to 400 T/m.

The twisted posture of the electrically conductive yarn 33 may be any type such as a single twist yarn (i.e., a yarn formed by pulling one or more yarns in parallel and twisting them together), a plied yarn (i.e., a yarn formed by pulling two or more single loose twist yarns or single soft twist yarns in parallel and twisting them together in a direction opposite to the direction of twists of the single twist yarns), a Koma twist yarn (i.e., a yarn formed by pulling two or more hard twist yarns in parallel and twisting them together in a direction opposite to the direction of twists of the single twist yarns), or a corkscrew twist yarn (i.e., a yarn formed by pulling a thick single twist yarn and a thin non-twist yarn in parallel and twisting them together in a direction opposite to the direction of twist of the single twist yarn). In addition, the directions of twists of a plurality of yarns included in the electrically conductive yarn may be all equal.

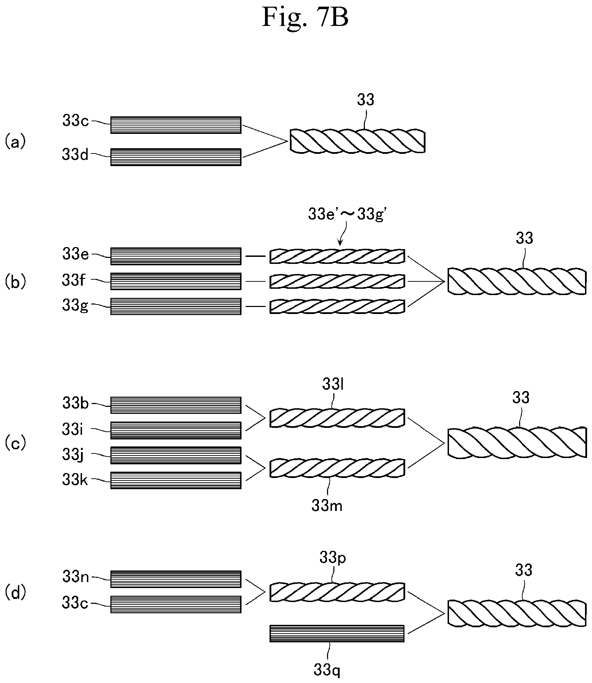

Figs. 7B are schematic views of some specific examples of the aforementioned twisted posture of the electrically conductive yarn 33. Fig. 7B(a) illustrates a single twist of two yarns, Fig. 7B(b) illustrates a Koma twist of three yarns, Fig. 7B(c) illustrates a twist of four yarns, and Fig. 7B(d) illustrates a corkscrew twist. As understood from the drawings, when the electrically conductive yarn 33 is formed by twisting a plurality of yarns in multiple stages, reversing the directions of twists of some of the yarns will prevent the yarns from becoming loose when they are conveyed on a machine such as a sewing machine, and thus, the yarns can be sent stably. In particular, when sewing is performed with a machine, such as a sewing machine, the outermost layer of the yarn is ideally a Z-twist yarn layer.

As described above, when the electrically conductive yarn 33 is formed by twisting the metallic yarn 33a and the non-metallic yarn 33b together, electrical conductivity significantly improves in comparison with conventional electrically conductive yarns formed by plating non-metallic yarns with metal. Further, since the metal wires can have many points of contact therebetween, it is possible to significantly suppress an increase in the resistance even when the metal wires deform or when one of the metal wires breaks. Accordingly, such an electrically conductive yarn can also be used for a communication scheme like I²C communication that uses digital sensors, though it has been conventionally difficult.

The aforementioned I²C (Inter-Integrated Circuit) communication is a communication scheme for performing serial communication with a device coupled over a short distance on the same substrate, for example, at high speed such as 100kbps or 400kbps, and is a scheme that realizes high-speed communication with mainly an EEPROM memory IC. In the I²C communication, high-speed communication should be performed. Therefore, wires with sufficiently high electrical conductivity should be used. In this point, metal wires such as copper wires are used as the stretchable wires used in this embodiment as described previously. Therefore, in comparison with conventional electrically conductive yarns formed by plating chemical fibers with metal, electrical conductivity significantly improves, and sufficient electrical conductivity for performing I²C communication can be secured.

To further increase the stability of the digital sensors, capacitors for preventing voltage drops can be used to stabilize the amounts of current supplied to the sensors. The capacitance of the capacitors is preferably in the range of 10 to 100 µF, or more preferably, in the range of 0.1 to 10 µF.

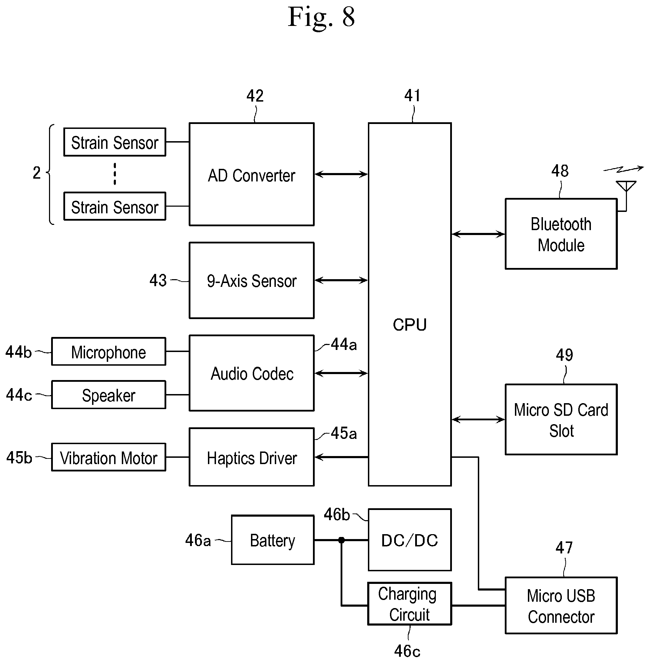

Next, the controller 4 used for the sensor system according to an embodiment of the present invention will be described with reference to Fig. 8. Fig. 8 is a block diagram illustrating the configuration of the controller 4 used for the sensor system according to this embodiment. It should be noted that Fig. 8 illustrates a case where strain sensors are used as examples of the sensors 2.

As illustrated in Fig. 8, the controller 4 has a CPU that receives measurement results of the sensors 2 and processes them. Signals detected with the sensors 2 (i.e., strain sensors) are converted by the AD converter 42 and are transmitted to the CPU 41.

The controller 4 also has a 9-axis sensor 43. The 9-axis sensor 43 includes, for example, a 3-axis gyroscope sensor that detects angular velocity (i.e., rotating speed), a 3-axis accelerometer that detects acceleration, and a 3-axis terrestrial magnetism sensor that detects terrestrial magnetism to detect the absolute direction. When the 9-axis sensor 43 functions, rotating motions or motions in the horizontal and vertical directions of the wearer can be detected.

The controller 4 also includes an audio codec 44a that encodes or decodes sound information. When the wearable device is provided with a microphone 44b, the audio codec 44a encodes voice, breath, and the like of the wearer collected via the microphone 44b, and sends them as data to the CPU. In addition, when the wearable device is provided with a speaker 44c, the audio codec 44a decodes various data such as a traveling distance and elapsed time, into sound data from the side operating the CPU, and sends it to the wearer via the speaker 44c.

When the wearable device is provided with a vibration motor 45b, the controller 4 also includes a haptics driver 45a that controls so-called tactile feedback. The haptics driver 45a measures, for example, the heart rate of the wearer via the vibration motor 45b. The vibration motor 45b for the wearer includes an eccentric motor, a linear vibrator, or a piezoelectric element, for example, and the haptics driver 45a includes a driver IC therefor.

The controller 4 also includes a detachable battery 46a and a DC/DC down-converter 46b that is coupled to the battery 46a and converts high voltage into low voltage. The battery 46a is coupled to a Micro USB connector 47 via a charging circuit 46c. The battery 46a can be charged both when it is mounted on the controller 4 and detached from the controller 4.

The controller 4 also includes a Bluetooth (registered trademark) module 48. The processing results of the CPU 41 can be sent to the outside via the module 48. Instead of or in addition to the Bluetooth module 48, a recording medium slot (i.e., a Micro SD card slot 49 in Fig. 8) may be provided so that data can be directly recorded on the recording medium. Although Fig. 8 illustrates Bluetooth (registered trademark) as a means of communication with the outside, the means of communication is not limited thereto, and communication standards such as Zigbee (registered trademark) and Wi-Fi (registered trademark), can also be used. Further, wire communication can also be used.

Although the embodiments of the present invention have been described with reference to the drawings, the present invention is not limited thereto, and any change in design is possible within the spirit and scope of the invention, with regard to the number of the sensors 2 arranged, the arrangement (e.g., bilaterally symmetrical/asymmetrical arrangement) of the wires 3, the number of the sensors 2 coupled to a single wire 3, and the shape and configuration of the controller 4.

### Reference Signs List

1 Fabric
1b Back side
1f Front side
2 Sensor
3 Stretchable wire
4 Controller
5 Cavity
6 Paper pattern
7 Cavity
111 Left shoulder portion
11r Right shoulder portion
121 Outer side portion of left arm
12r Outer side portion of right arm
131 Inner side portion of left arm
13r Inner side portion of right arm
141 Left flank side portion
14r Right flank side portion
31 Short-circuit prevention layer
31a, 31c, 32a Bonding layer
31b, 32b Insulating layer
32 Stretchable film
33 Electrically conductive yarn
33a Metallic yarn
33b Non-metallic yarn
33c to 33q Yarn
34 Wire layer
35 Protective layer
40 Connector portion
41 CPU
42 AD converter
43 9-axis sensor
44a Audio codec
44b Microphone
44c Speaker
45a Haptics driver
45b Vibration motor
46a Battery
46b DC/DC
46c Charging circuit
47 Micro USB connector
48 Bluetooth module
49 Micro SD card unit
100 Wearable device
OF Front open portion
OFl Left front open portion
OFr Right front open portion
OP Opening
61 Paper pattern body
62, 63 Paper pattern flank portion
6111, 611r, 6121, 612r, 613lf, 613lb, 613rf, 613rb, 614lf, 614lb, 614rf, 614rb Paper pattern portion

## Claims

1. An upper-body wearable device having wires disposed on a fabric having a front side and a back side, the wearable device comprising:
electronic components, as least one of which being disposed on the front side and on the back side, respectively; and
a single controller configured to control the electronic components,
wherein:
a boundary between the front and back sides of the fabric includes a first seamed portion and a second seamless portion, and
one or more of the wires are disposed on the front and back sides across the second seamless portion on the boundary so as to electrically couple the electronic components disposed on the front and back sides to the controller.

2. The wearable device according to claim 1, wherein each electronic component includes at least one of a sensor, an actuator, or a light source.

3. The wearable device according to claim 1 or 2, wherein each wire is a stretchable wire, the stretchable wire including a stretchable substrate and an electrically conductive yarn arranged in the stretchable substrate in a zigzag pattern.

4. The wearable device according to any one of claims 1 to 3,
wherein:
one of the front side or the back side of the wearable device includes a front open portion, the front open portion dividing the one of the front side or the back side into a right half side and a left half side, and
the controller is disposed on the right half side and the left half side across the front open portion.

5. A paper pattern for producing an upper-body wearable device, the wearable device including a fabric having a front side and a back side, wires disposed on the fabric, electronic components at least one of which being disposed on the front side and on the back side, and a single controller configured to control the electronic components, one or more of the wires being disposed on the front and back sides across a boundary between the front and back sides so as to electrically couple the electronic components disposed on the front and back sides to the controller,
wherein:
at least a part of the paper pattern corresponding to the boundary between the front and back sides of the fabric is continuous.

6. The paper pattern according to claim 5, further comprising a pair of paper pattern side portions corresponding to flank side faces of the fabric.
